# EUROPEAN PATENT SPECIFICATION

(11) **EP 2 698 149 B1**
(45) Date of publication and mention of the grant of the patent: **27.09.2017**
(21) Application number: 13180739.8
(22) Date of filing: 16.08.2013
(51) Int. Cl.: A61K 9/00, A61K 31/155, A61K 31/192, A61P 1/02

(54) **Topical Aqueous Pharmaceutical Compositions of Flurbiprofen and Chlorhexidine**
Topische wässrige pharmazeutische Zusammensetzungen enthaltend Flurbiprofen und Chlorhexidin
Compositions pharmaceutiques topiques aqueuses contenant du flurbiprofène et de la chlorhexidine

(30) Priority: 17.08.2012 TR 201209600
(43) Date of publication of application: 19.02.2014
(60) Divisional application: 15151700.0
(73) Proprietor: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- WO-A1-95/04520
- WO-A1-2011/067693
- AT-U1- 11 910
- JP-K1- H08 268 854
- US-A- 4 025 616

## Description

### Field of the Invention

The present invention relates to stable topical aqueous pharmaceutical compositions comprising flurbiprofen or pharmaceutically acceptable salts thereof and chlorhexidine or salts thereof. More particularly, the present invention relates to a stable oral topical aqueous pharmaceutical composition of this combination having desired levels of dissolution rate, solubility, enhanced taste and absorption from mucosal surface, comprising mint oil or eucalyptus oil and wherein the pH of the solution is between 6 to 7.

### Background of the Invention

Flurbiprofen is a well-known non-steroidal anti-inflammatory drug which has analgesic and anti-inflammatory activities. It is used in muscle-skeletal and joint disorders such as ankylosing spondylitis, osteoarthritis and rheumatoid arthritis, in soft-tissue disorders such as sprains and strains and for postoperative pains. Its chemical structure is illustrated with Formula I given below.

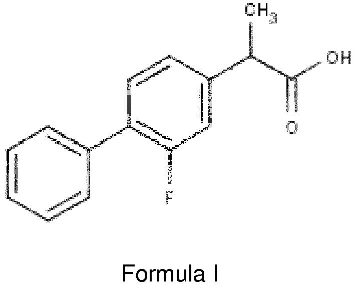

Flurbiprofen is mostly administrated orally. One disadvantage of the oral administration of flurbiprofen comprising compositions is that the patient is likely to experience unpleasant side effects, including gastrointestinal irritation. The use of flurbiprofen in treating local pains and inflammations may cause a problem especially for those who have gastrointestinal system disorders. It is possible to develop various locally-administrable topical forms of flurbiprofen, in order to avoid the systemic side-effects thereof.

Another disadvantage of flurbiprofen is that it is practically insoluble in water. This makes it difficult to prepare its aqueous solutions.

Chlorhexidine is an antiseptic and disinfectant effective against a wide variety of bacteria, fungi and some viruses. It is used clinically in various preparations for disinfecting purposes such as oral rinses and skin cleansers. It is often used in mouthwash designed for reducing dental plaque and oral bacteria and to treat and prevent gingivitis. Its chemical structure is illustrated with Formula II given below.

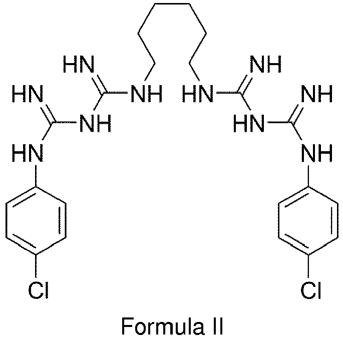

Chlorhexidine is a strong base and is most stable in the form of salts such as the digluconate and dihydrochloride. The most commonly used derivative is the digluconate salt because of its higher water-solubility.

Chlorhexidine and its derivatives have the disadvantage that they stain the teeth with repeated use and have a bitter taste. Thus there is a need to increase the water solubility of chlorhexidine and its derivatives and to mask the bitter taste of these compounds so that they can be more incorporated into the aqueous based compositions to decrease the level of staining the teeth significantly and find greater acceptability to the user.

PCT patent application WO 95/04520 describes the use of chlorhexidine and a non-steoridal anti-inflammatory agent for the treatment of epidermal and muco-epidermal tissue. Non-steoridal anti-inflammatory agent is stated to include flurbiprofen. However this document does not describe a specific formulation for this combination.

A Canadian patent application CA 2275049-A1 describes a needle-like device adapted for insertion in a root canal and pulp chamber of a tooth of the patient, comprising chlorhexidine and another biological active agent wherein the biological active agent consisting of flurbiprofen and some other agents. However it does not describe a topical aqueous formulation of this combination.
A Japanese patent document JP 8268854 discloses a composition containing an acidic non-steroidal anti-inflammatory agent, a cationic antimicrobial agent, a tetracy cline antibiotic and an inorganic acid for use in oral cavities. Flurbiprofen and chlorhexidine are stated for non-steroidal anti-inflammatory agent and cationic antimicrobial agent respectively. This composition further comprises an antibiotic and an acid. This document does not disclose the specific combination of flurbiprofen and chlorhexidine. In addition to this, the composition described in JP 8268854 contains an acid which decreases the solubility of flurbiprofen and thus dissolution and the extent of penetration into the muco-epidermal and epidermal tissue decreases.

Accordingly, for the treatment of periodontal diseases and muco-epidermal and epidermal inflammation and infection there arises the need of an oral topical aqueous composition comprising flurbiprofen and chlorhexidine which optimizes the delivery of them through the oral mucosal surface.

### Summary of the Invention

It is therefore an object of the present invention to provide an oral topical aqueous pharmaceutical composition of flurbiprofen or pharmaceutically acceptable salts thereof and chlorhexidine or pharmaceutically acceptable salts thereof having desired levels of dissolution rate, solubility enhanced taste and absorption from mucosal surface.

The inventors of the present invention have discovered that by using an alkalizing agent the pH of the solution is adjusted to pH 6-7 thus the solubility of flurbiprofen is increased and absorption through the oral mucosal surface achieved to be at the desired levels. Therefore it is another object of the invention to provide an oral topical aqueous pharmaceutical composition of flurbiprofen or pharmaceutically acceptable salts thereof and chlorhexidine or pharmaceutically acceptable salts wherein the pH of the solution is between 6 and 7.

In addition to this the inventors of the present invention have discovered that mint oil or eucalyptus oil not only masks the bitter taste of flurbiprofen and chlorhexidine but also increase the dissolution rate, solubility and improve the clearness of the solution. Thus it is another object of the invention to provide an oral topical aqueous pharmaceutical composition of this combination comprising mint oil or eucalyptus oil and wherein the pH of the solution is between 6 to 7.

The invention further provides a treatment of periodontal diseases and non-specific epidermal and muco-epidermal infections and inflammation using such preparations.
Another object of the present invention is to provide easily applicable oral topical aqueous pharmaceutical compositions of flurbiprofen or pharmaceutically acceptable salts thereof and chlorhexidine or salts thereof having desired levels of dissolution rate, solubility and having improved taste and preventing the staining of the teeth caused by the regular use of chlorhexidine.
A further object of the present invention is to obtain a stable topical aqueous pharmaceutical composition of flurbiprofen and chlorhexidine during the shelf-life and to exhibit high safety when applied to the oral mucosal surface.
A further object of the present invention is to obtain a formulation with local anesthetic effect, with the mint oil or eucalyptus oil used in said combination stimulating the receptors by which the cold sensation is perceived.
Further advantages and embodiments of the present invention will become apparent from the following description

### Detailed Description of Invention

According to the present invention, a novel oral topical aqueous pharmaceutical composition with anti-inflammatory and antiseptic activities is obtained surprisingly, which is having desired levels of dissolution rate, solubility, enhanced taste and rapidly absorbed from the oral mucosa. Further, this formulation does not cause the staining of the teeth which is an unwanted effect of chlorhexidine in oral aqueous preparations.
Flurbiprofen useful in accordance with this invention includes the pharmaceutically acceptable salts and esters of flurbiprofen, and further includes the conventionally used racemic mixture which comprises the S- and R- enantiomers of flurbiprofen. The topical aqueous pharmaceutical compositions of the invention comprise from 0.01 to 4,75% flurbiprofen, preferably from 0,05 to 2.5%, more preferably from 0.15 to 1.25% by weight of the total composition.

The topical aqueous pharmaceutical compositions of the invention comprise from 0.01 to 4.0% chlorhexidine or salts and esters thereof, preferably from 0.02 to 3.0%, more preferably from 0.08 to 2.0% by weight of the total composition.

The topical aqueous pharmaceutical compositions of the invention comprise mint oil or eucalyptus oil, preferably mint oil, from 0.001 to 2.75%, preferably from 0.05 to 1.0%, more preferably from 0.06 to 0.25% by weight of the total composition. Mint oil and eucalyptus oil help the composition of the present invention to improve and enhance the penetrating and spreading properties through the oral mucosal surface. They also help to increase the solubility of the flurbiprofen and chlorhexidine and thus a homogenous and clear solution is obtained. Accordingly, mint oil or eucalyptus oil is used as a flavoring agent and masks the bitter taste of chlorhexidine and flurbiprofen. When other flavoring agents such as menthol are used an unclear and blurry solution is obtained. The present inventors discovered that using mint oil or eucalyptus oil as a taste enhancer solves this problem and a clear solution is obtained as desired.

Furthermore mint oil or eucalyptus oil used in the formulation gives anesthetic effect at the side of administration as a result of stimulating the receptors by which cold sensation is perceived. Mint oil or eucalyptus oil when used with flurbiprofen and chlorhexidine, provides significant relief of pain associated with periodontal disease and non-specific epidermal and muco-epidermal infections. Also mint oil or eucalyptus oil help to mask the bad odour of the other excipients to obtain a good patient compliance when applying inside the mouth.

The topical aqueous pharmaceutical compositions of the invention comprise an alkalizing agent to adjust the pH of the solution to pH 6-7. Adjusting the pH of the solution to about 6 to 7 pH helps to enhance the dissolution, extent of penetration into muco-epidermal and epidermal tissue and bioavailability. It also helps to increase the solubility of flurbiprofen which is otherwise insoluble in water thus making the aqueous formulations of it difficult. Adjusting the pH to about 6-7 both helps to improve the solubility of flurbiprofen and chlorhexidine and increase the rate of absorption of them from the oral mucosal surface.

Suitable alkalizing agents comprise but not limited to sodium hydroxide, calcium hydroxide, diethanolamine, monoethanolamine, potassium bicarbonate, potassium citrate, potassium hydroxide, sodium bicarbonate, sodium borate, sodium carbonate, sodium citrate dehydrate, triethanolamine and mixtures thereof. Preferably alkalizing agent is sodium hydroxide. Suitable alkalizing agents are preferably from 0,1 to 2,5%, more preferably from 0,12 to 2,2% by weight of the total composition.

The pharmaceutical compositions according to the present invention may also comprise one or more pharmaceutically acceptable excipients. Such proper pharmaceutically acceptable excipients comprise, but are not limited to surfactants, viscosity enhancers, flavoring agents, sweetening agents, solvents, and their mixtures. The pharmaceutical compositions according to the present invention further comprise a dye optionally.
Suitable surfactants may comprise but not limited to polyoxyethylene castor oil derivatives, polysorbate, polyexyethylene stearates, polyoxylglycerides, glyceryl monooleate, sorbic acid, butylparaben, phospholipids and mixtures thereof. Preferably the surface active agent is polyoxyethylene castor oil derivatives, more preferably polyoxyl 40 hydrogenated castor oil. The amount of polyoxyethylene castor oil derivative is from 0,1 to 5,0%, preferably from 0,5 to 2,5% by weight of the total composition. These amounts of polyoxyethylene castor oil derivatives improve the spreading properties and minimize drying of the aqueous compositions of the present invention when it is applied inside of the mouth via spray, mouth rinse or mouthwash.

Suitable viscosity enhancers may comprise but not limited to glycerin, sodium carboxyl methyl cellulose, dextran, cellulose and derivatives, chitosan, carbomer and mixtures thereof. Preferably the viscosity enhancer is glycerin and the amount is from 5.0 to 25.0%, preferably from 10.0 to 20%, more preferably from 12.0 to 18.0% by weight of the total composition. Glycerin has also a stabilization effect when it is used in these amounts and helps the formulation to be stable over the shelf life.

Suitable flavoring agents may comprise but not limited to mint oil, eucalyptus oil, carnation oil, ginger oil, lavender oil, sweet orange oil and their mixtures.

Suitable sweetening agents may comprise but not limited to sorbitol, saccharin, saccharin sodium, aspartame, fructose, isomalt, maltitol, maltose, mannitol, sucrose, xylitol, glycerin and their mixtures, and the amount is from 0,1 to 15,0%, preferably 5.0 to 12.0% by weight of the total composition.

Suitable solvents may comprise but not limited to ethyl alcohol, polyethylene glycol, propylene glycol, isopropyl alcohol, glycerin, sorbitol, purified water and mixtures thereof. Preferably ethyl alcohol, sorbitol, glycerin and purified water are used. Ethyl alcohol is also utilized as a microbiological preservative. Suitable solvents are used in an amount of from 5 to 40%, preferably from 10 to 30% by weight of the total composition.

Suitable dyes may comprise but not limited to Patent Blue, quinoline yellow, orange G and mixtures thereof. Preferred dye is patent blue.

The pharmaceutical composition according to the present invention comprises most preferably on a weight basis:
(i) between 0,05 to 2,5% flurbiprofen or pharmaceutically acceptable salts thereof;
(ii) between 0.02 to 3.0% chlorhexidine or salts thereof;
(iii) between 0,05 to 1,0% mint oil or eucalyptus oil;
(iv) between 0,1 to 2,5% sodium hydroxide
(v) between 5 to 20 % sorbitol;
(vi) between 0,1 to 0,5% saccharine sodium;
(vii) between 5 to 25% glycerin;
(viii) between 0.1 to 5% polyoxyl 40 hydrogenated castor oil;
(ix) between 5 to 15 % ethyl alcohol

The pharmaceutical composition according to the present invention provides oral topical aqueous pharmaceutical compositions of flurbiprofen or a pharmaceutically acceptable salt thereof and chlorhexidine or salts thereof. The topical compositions of the invention may be in the form of mouthwash, mouth rinse and spray.

Accordingly, the present invention may be used for treating periodontal diseases and non-specific epidermal and muco-epidermal infections and inflammations.

This invention is further defined by reference to the following examples. Although the examples are not intended to limit the scope of the present invention, they should be considered in the light of the description detailed above.

### Example 1

### Preparation of the oral topical aqueous solution

Flurbiprofen was dissolved in ethyl alcohol and stirred for 10 minutes. Sodium hydroxide solution was added to the mixture. Glycerin, sorbitol, polyoxyl 40 hydrogenated castor oil, purified water and chlorhexidine gluconate solution was added and stirred for 15 minutes. Saccharine sodium was dissolved in purified water and added to the solution. The solution was stirred for 10 minutes. Patent blue was added and stirred for 10 minutes. Mint oil was added and stirred for 10 minutes. The pH of the solution was adjusted to 6.7 by adding sodium hydroxide to the solution. Purified water was added to the solution to make the volume of the solution 200 ml.

**Final composition:**

| | |
|---|---|
| Flurbiprofen | 0.50 g |
| Chlorhexidine gluconate | 0.24 g |
| Sorbitol | 20.0 g |
| Saccharine sodium | 0.30 g |
| Glycerin | 30.0 g |
| Polyoxyl 40 hydrogenated castor oil | 2.0 g |
| Ethyl alcohol | 16.0 g |
| Patent blue | 0.0008 g |
| Eucalyptus oil | 0.15 g |
| Sodium hydroxide | 0.32 g |
| Purified water, sufficient for | 200 mL |

### Example 2

### Preparation of the oral topical aqueous solution

Following formulation is prepared as in the formulation method in Example 1.

| | |
|---|---|
| Flurbiprofen | 0.50 g |
| Chlorhexidine gluconate | 0.24 g |
| Sorbitol | 20.0 g |
| Saccharine sodium | 0.30 g |
| Glycerin | 30.0 g |
| Polyoxyl 40 hydrogenated castor oil | 2.0 g |
| Ethyl alcohol | 16.0 g |
| Patent blue | 0.0008 g |
| Mint oil | 0.15 g |
| Sodium hydroxide | 0.32 g |
| Purified water, sufficient for | 200 mL |

## Claims

1. An oral topical aqueous pharmaceutical composition comprising:
(i) flurbiprofen or pharmaceutically acceptable salts thereof;
(ii) chlorhexidine or pharmaceutically acceptable salts thereof;
wherein the pH of the composition is between 6 and 7; and said composition further comprises mint oil in an amount from 0,001% to 2.75% by weight of the total composition.

2. The composition according to claim 1 comprising on a weight basis,
(i) between 0,01 to 4,75% flurbiprofen or pharmaceutically acceptable salts thereof;
(ii) between 0.01 to 4.0% chlorhexidine or salts thereof.

3. The composition according to claims 1 to 2 comprising on a weight basis,
(i) between 0,01 to 4,75% flurbiprofen or pharmaceutically acceptable salts thereof;
(ii) between 0.01 to 4.0% chlorhexidine or salts thereof;
(iii) sodium hydroxide in a sufficient amount to adjust the pH of the solution to between 6 and 7.

4. The composition according to claims 1 to 3, further comprising at least one solvent in an amount of from 5 to 40 % by weight of the total composition.

5. The composition according to claim 4, wherein the solvent is selected from ethyl alcohol, glycerin and sorbitol.

6. The composition according to claims 1 to 5, further comprising at least one sweetening agent in an amount of from 0.1 to 15.0% by weight of the total composition.

7. The composition according to claim 6, wherein the sweetening agent is selected from saccharine sodium and sorbitol.

8. The composition according to claims 1 to 7, further comprising at least one surfactant in an amount of from 0.1 to 5.0% by weight of the total composition.

9. The composition according to claim 8, wherein the surfactant is polyoxyl 40 hydrogenated castor oil.

10. The composition according to any of the preceding claims, comprising on a weight basis:
(i) between 0,05 to 2,5% flurbiprofen or pharmaceutically acceptable salts thereof;
(ii) between 0.02 to 3.0% chlorhexidine or salts thereof;
(iii) between 0,1 to 2,5% sodium hydroxide
(iv) between 5 to 20 % sorbitol;
(v) between 0,1 to 0,5% saccharine sodium;
(vi) between 5 to 25% glycerin;
(vii) between 0.1 to 5% polyoxyl 40 hydrogenated castor oil;
(viii) between 5 to 15 % ethyl alcohol

11. The composition according to any one of the preceding claims, wherein said composition is in the form of a mouthwash, mouth rinse or spray.

12. The composition according to any one of the preceding claims, for use in the treatment of periodontal diseases and non-specific epidermal and muco-epidermal infections and inflammation.

## Patentansprüche

1. Eine orale, topische, wässrige, pharmazeutische Zusammensetzung umfassend:
(i) Flurbiprofen oder pharmazeutisch verträgliche Salze davon;
(ii) Chlorhexidin oder pharmazeutisch verträgliche Salze davon;
(iii) wobei der pH der Zusammensetzung zwischen 6 und 7 ist; und die Zusammensetzung des Weiteren Minzöl in einer Menge von 0,001 Gew% bis 2,75 % bezogen auf das Gewicht der Gesamtzusammensetzung umfasst.

2. Zusammensetzung gemäß Anspruch 1, umfassend auf Gewichtsbasis,
(i) zwischen 0,01 bis 4,75 % Flurbiprofen oder pharmazeutisch verträgliche Salze davon;
(ii) zwischen 0,01 bis 4,0 % Chlorhexidin oder Salze davon.

3. Zusammensetzung gemäß der Ansprüche 1 bis 2, umfassend auf Gewichtsbasis,
(i) zwischen 0,01 bis 4,75 % Flurbiprofen oder pharmazeutisch verträgliche Salze davon;
(ii) zwischen 0,01 bis 4,0 % Chlorhexidin oder Salze davon;
(iii) Natriumhydrochid in einer ausreichenden Menge, um den pH der Lösung auf 6 bis 7 einzustellen.

4. Zusammensetzung gemäß der Ansprüche 1 bis 3, des Weiteren mindestens ein Lösungsmittel in einer Menge von 5 bis 40 % bezogen auf das Gewicht der Gesamtzusammensetzung umfassend.

5. Zusammensetzung gemäß Anspruch 4, wobei das Lösungsmittel ausgewählt ist aus Ethylalkohol, Glyzerin und Sorbitol.

6. Zusammensetzung gemäß der Ansprüche 1 bis 5, des Weiteren mindestens ein Süßungsmittel in einer Menge von 0,1 bis 15,0 % bezogen auf das Gewicht der Gesamtzusammensetzung umfassend.

7. Zusammensetzung gemäß Anspruch 6, wobei das Süßungsmittel ausgewählt ist aus Sodiumsaccharin und Sorbitol.

8. Zusammensetzung gemäß der Ansprüche 1 bis7, des Weiteren mindestens ein oberflächenaktives Mittel in einer Menge von 0,1 bis 5,0 % bezogen auf das Gewicht der Gesamtzusammensetzung umfassend.

9. Zusammensetzung gemäß Anspruch 1, wobei das oberflächenaktive Mittel Polyoxyl 40 hydriertes Rizinusöl ist.

10. Zusammensetzung gemäß einem der vorhergehenden Ansprüche, umfassend auf Gewichtsbasis:
(i) zwischen 0,05 bis 2,5 % Flurbiprofen oder pharmazeutisch verträgliche Salze davon;
(ii) zwischen 0,02 bis 3,0 % Chlorhexidin oder Salze davon;
(iii) zwischen 0,1 bis 2,5 % Natriumhydroxid,
(iv) zwischen 5 bis 20 % Sorbitol,
(v) zwischen 0,1 bis 0,5 % Natriumsaccharin;
(vi) zwischen 5 bis 25 % Glyzerin;
(vii) zwischen 0,1 bis 5,0 % Polyoxyl 40 hydriertes Rizinusöl;
(viii) zwischen 5 bis 15 % Ethylalkohol.

11. Zusammensetzung gemäß einem der vorhergehenden Ansprüche, wobei die Zusammensetzung in der Form eines Mundwassers, Mundspülung oder Sprays vorliegt.

12. Zusammensetzung gemäß einem der vorhergehenden Ansprüche zur Anwendung in der Behandlung von Parodontalerkrankungen und nicht spezifischen epidermischen und mucoepidermischen Infektionen und Entzündungen.

## Revendications

1. Composition pharmaceutique aqueuse topique orale, comprenant :
(i) du flurbiprofène ou des sels pharmaceutiquement acceptables de celui-ci ;
(ii) de la chlorhexidine ou des sels pharmaceutiquement acceptables de celle-ci ;
dans laquelle le pH de la composition est entre 6 et 7 ; et ladite composition comprend en outre de l'huile de menthe dans une quantité de 0,001 % à 2,75 % en poids de la composition totale.

2. Composition selon la revendication 1, comprenant, sur une base en poids,
(i) entre 0,01 et 4,75 % de flurbiprofène ou de sels pharmaceutiquement acceptables de celui-ci ;
(ii) entre 0,01 et 4,0 % de chlorhexidine ou de sels celle-ci.

3. Composition selon l'une des revendications 1 et 2, comprenant, sur une base en poids,
(i) entre 0,01 et 4,75 % de flurbiprofène ou de sels pharmaceutiquement acceptables de celui-ci ;
(ii) entre 0,01 et 4,0 % de chlorhexidine ou de sels de celle-ci ;
(iii) de l'hydroxyde de sodium dans une quantité suffisante pour ajuster le pH de la solution à entre 6 et 7.

4. Composition selon l'une des revendications 1 à 3, comprenant en outre au moins un solvant dans une quantité de 5 à 40 % en poids de la composition totale.

5. Composition selon la revendication 4, dans laquelle le solvant est choisi parmi l'alcool éthylique, le glycérol et le sorbitol.

6. Composition selon l'une des revendications 1 à 5, comprenant en outre au moins un agent édulcorant dans une quantité de 0,1 à 15,0 % en poids de la composition totale.

7. Composition selon la revendication 6, dans laquelle l'agent édulcorant est choisi parmi la saccharine sodique et le sorbitol.

8. Composition selon l'une des revendications 1 à 7, comprenant en outre au moins un tensio-actif dans une quantité de 0,1 à 5,0 % en poids de la composition totale.

9. Composition selon la revendication 8, dans laquelle le tensio-actif est l'huile de ricin hydrogénée polyoxylée 40.

10. Composition selon l'une quelconque des revendications précédentes, comprenant, sur une base en poids :
(i) entre 0,05 et 2,5 % de flurbiprofène ou de sels pharmaceutiquement acceptables de celui-ci ;
(ii) entre 0,02 et 3,0 % de chlorhexidine ou de sels de celle-ci ;
(iii) entre 0,1 et 2,5 % d'hydroxyde de sodium ;
(iv) entre 5 et 20 % de sorbitol ;
(v) entre 0,1 et 0,5 % de saccharine sodique ;
(vi) entre 5 et 25 % de glycérol ;
(vii) entre 0,1 et 5 % d'huile de ricin hydrogénée polyoxylée 40 ;
(viii) entre 5 et 15 % d'alcool éthylique.

11. Composition selon l'une quelconque des revendications précédentes, dans laquelle ladite composition se présente sous la forme d'un bain de bouche, d'un rince-bouche ou d'une pulvérisation.

12. Composition selon l'une quelconque des revendications précédentes, pour une utilisation dans le traitement de maladies périodontiques et d'infections et inflammation épidermiques et muco-épidermiques non spécifiques.
